# EUROPEAN PATENT APPLICATION

(11) **EP 2 075 340 A2**
(43) Date of publication of application: **01.07.2009**
(21) Application number: 09003768.0
(22) Date of filing: 30.09.2004
(51) Int. Cl.: C12Q 1/68, A61K 48/00

(54) **Gene modulation by RB2/p 130 expression**

(30) Priority: 30.09.2003 US 507677 P
(62) Divisional of application: 04789421.7
(71) Applicant: Sbarro Health Research Organization, Radnor, PA 19087 (US)
(72) Inventor: Giordano, Antonio, Radnor, PA 19087-4686 (US)
(74) Representative: Vossius & Partner

(57) **Abstract**

The present invention discloses a set of molecular signatures modulated by RB2/p130 for lung cancer cells and a protein encoded by the system to modulate a gene or gene expression pattern in lung cancer cells of mammals.

## Description

This application claims the benefit of U.S. Provisional Application No. 60/507,677 filed September 30, 2003, the text of which application is hereby incorporated by reference in its entirety.

### FIELD OF THE INVENTION

The present invention relates to the fields of oncology and molecular biology. More particularly the invention relates to RB2/p130 modulated molecular signatures in lung cancer cells and the diagnosis and prognosis of lung cancer using RB2/p130 modulated molecular signatures. The present invention also relates to strategies for the use of RB2/p130 to regulate the expression of gene products in lung cancer cells.

### BACKGROUND OF THE INVENTION

Citation or identification of any scientific reference in this application shall not be construed as an admission that such reference is available as prior art to the present invention.

Lung cancer is one of the leading causes of cancer death in the world. The high mortality rate for lung cancer probably results, at least in part, from the lack of standard clinical procedures for the diagnosis of the disease at early and more treatable stages compared to breast, prostate, and colon cancers (Wiest et al., 1997). There is also extremely poor prognosis associated with diagnosis of the disease, especially in advanced disease. The majority of bronchogenic carcinomas can be classified into four histological types: small cell lung carcinomas, adenocarcinomas, squamous cell lung carcinomas, and large cell carcinomas. Small cell lung carcinomas are a separate entity, whereas the behavior of the other three histological subtypes is similar, for this reason these are grouped within the non-small cell lung cancer (NSCLC) type. The NSCLC accounts for nearly 80% of lung malignant tumors and it is associated with a poor prognosis.

Early detection is difficult since clinical symptoms are often not seen until the disease has reached an advanced stage. Currently, diagnosis is aided by the use of chest x-rays, analysis of the type of cells contained in sputum and examination of the bronchial passages. Treatment regimens are determined by the type and stage of the cancer, and include surgery, radiation therapy and/or chemotherapy. Because of their lack of molecular specificity, these treatment regimens are not completely effective. For example, a major problem in the chemotherapy of cancers is the delivery of therapeutic agents, such as drugs, in sufficient concentrations to eradicate tumor cells while at the same time minimizing damage to normal cells. Thus, studies in many laboratories are directed toward the design of more specific systems such as antibodies, cytokines, and viruses for targeted delivery of genes into tumor cells. Because of their biospecificity, such systems could in theory deliver therapeutic agents to tumors.

Indeed, it is known in the art that lung cancer is the result of molecular changes in the cell, resulting in the deregulation of pathways which control normal cellular growth, differentiation, and apoptosis. Various genes such as protooncogenes and tumor suppressor genes are found to be mutated or have abnormal expression patterns in this disease. Also, the gene therapeutic potential of a number of genes in lung cancer has also been reported (see, for example, US Patents 6,663,856 and 6,797,702). If molecular markers that mediate potential therapeutic effects of genes used in gene therapy programs are available, it can facilitate the selection of the appropriate gene therapy to lung cancer thereby maximizing therapeutic efficacy and minimizing toxicity. Accordingly, there is a need in the art for identification of genes that are regulated by each of the known therapeutic genes for lung cancer so that the expression products can be used as molecular signatures for selecting an appropriate therapeutic gene for modulating the genes expressed in lung cancer cells. The present invention fulfills these needs and further provides other related advantages.

### SUMMARY OF THE INVENTION

In the present invention, a set of molecular signatures modulated by Rb2/p130 in lung cancer cells have been discovered. The identified molecular signatures or markers in a lung tissue sample provide a basis for the use of Rb2/p130 to modulate a gene or genes expressing the molecular signatures.

Accordingly, in a general aspect, the present invention provides a method for determining whether to use RB2/p130 (either a gene expression system or a protein encoded by the pRb2/p130 to modulate a gene or gene expression pattern in lung cancer cells of a mammalian test subject. The method involves providing molecular signatures modulated by RB2/p130 for lung cancer cells. The molecular signatures include expression products of one or more of the following genes: PCNA, MKK3, B-MYB, CCNF, BUB1B,PLK, NIK, KNSL2, PCSK7, CCNB2, GPRK6, HCFC1, PFAS, DNMT1, KPNA2, STK15, TIEG, BUB1 ELK1, UMPK, PMI, CAMKK2, GSK3B, HADHSC, POLD1, NOLI, EMK1, GRP-R, XRCC3, CHK, MAGEA3/6, PPM1G, TRAF5, ABCF2, TEAD4, PIM1, CCND1, CDR2, PSMB2 and RAF1. The method further involves determining gene or genes expressed in the lung cancer cells of the human test subject and using the RB2/p130 to modulate the gene or the gene expression pattern in the lung cancer cells of the mammalian test subject if it is found that the gene or genes expressed in the lung cancer cells of the mammalian test subject are the same as the one or more of the above listed genes. For example, the genes can be a set of genes such as B-MYB, PCSK7, STK15, ELK1, NOL1, MAGEA3/6, PIM1, CCND1, CDR2, and RAF1, all of which are associated with diseases. The mammal can be pre-treatment or post-treatment for a non-small cell lung cancer, the treatment being surgical operation, chemotherapy, radiation therapy and RB2/p130 gene therapy or combinations these treatments.

### BRIEF DESCRIPTION OF THE DRAWINGS

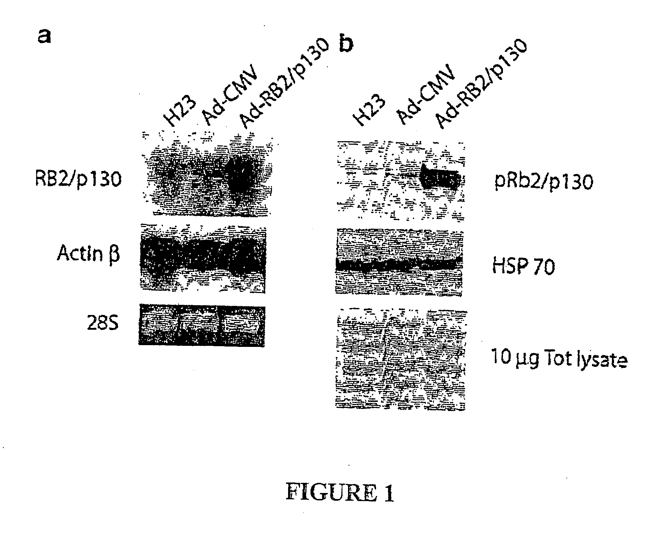

**Figure 1****.** Adenovirus-mediated overexpression of RB2/p130. Northern blot and Western blot analyses (a & b) of RB2/p130 in H23, H23-Ad-CMV and H23 Ad-CMV-RB2/p130 non small lung cancer cell line.

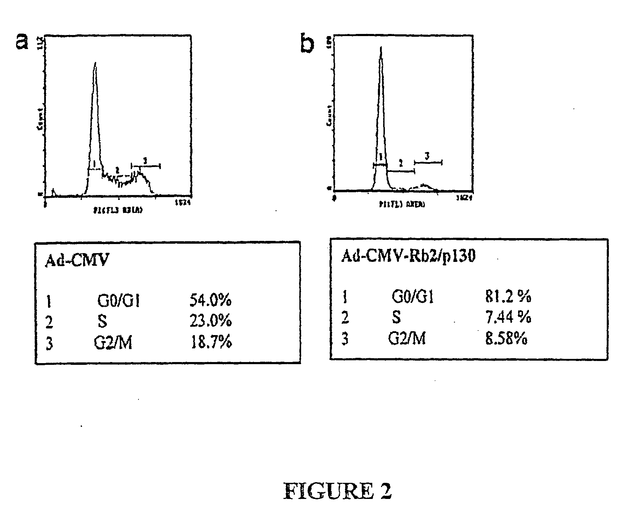

**Figure 2****.** Effects of pRb2/p130 enhanced expression in H23 cells. FACS analysis of H23-Ad-CMV (a) and H23-Ad-CMV-Rb2/p130 (b) infected cells. Rb2/p130 over-expression resulted in 81.2% of the cells accumulated in the G0/G1 phase of the cell cycle when compared to the empty adenovirus (54%). The analysis was performed in triplicates with comparable results.

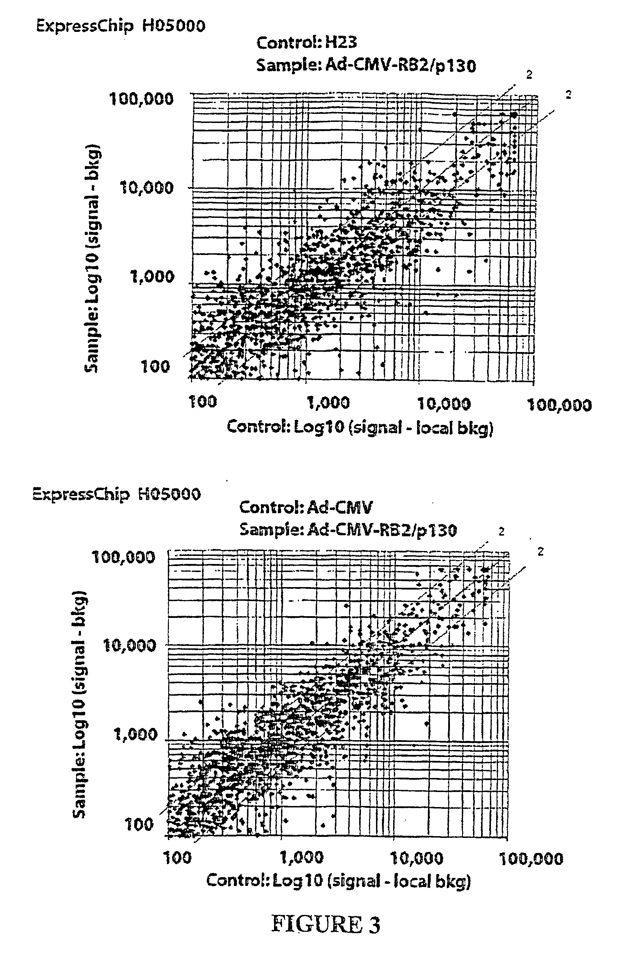

**Figure 3**. Global comparison of gene expression in H23 vs. H23-Ad-CMV and H23-Ad-CMV vs. H23-Ad-CMV-RB2 cells. Each dot corresponds to the Cy3 fluorescent intensity of one single element on the oligonucleotide microarray. A two-fold change in expression is indicated with parallel lines marked as 2.

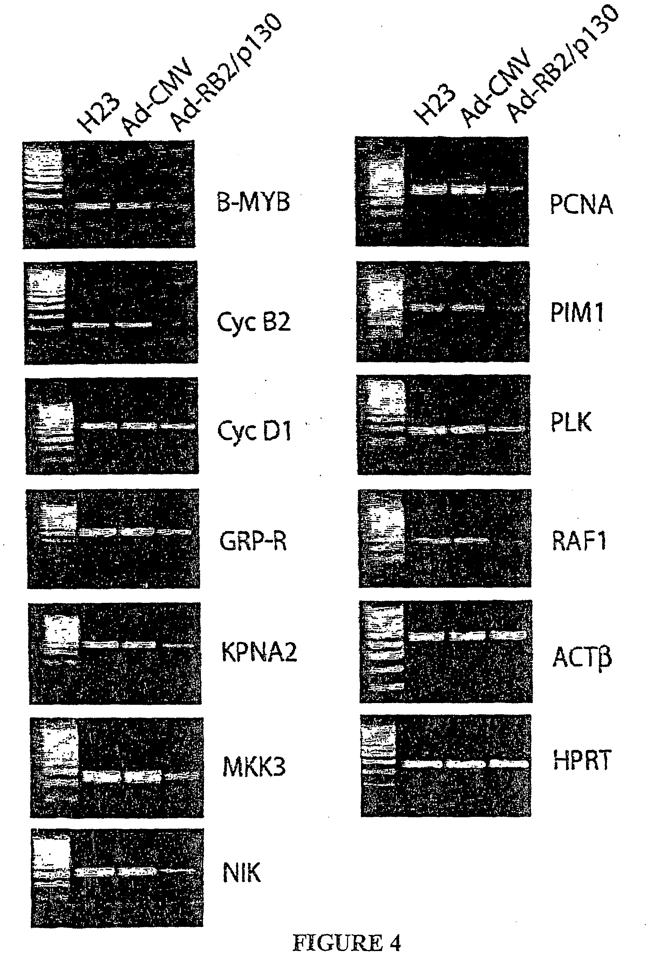

**Figure 4****.** Validation of oligonucleotide microarray results of 11 selected genes by semi quantitative RT-PCR. RT-PCRs were performed using DNAse treated total RNA of H23, H23-Ad-CMV and H23-Ad-CMV-Rb2/p130 non small lung cancer cell line. Amplified fragments of B-MYB (194 bp), Cyc B2 (217 bp), Cyc D1 (463 bp), GRPR (377 bp), KPNA2 (304 bp), MKK3 (219 bp), NIK (317 bp), PCNA (420 bp), PIM1 (324 bp), PLK (154 bp) and RAF1 (280 bp) genes are indicated. ACT-β (626 bp) and HPRT (349 bp) genes were used as internal controls and were amplified from the same samples.

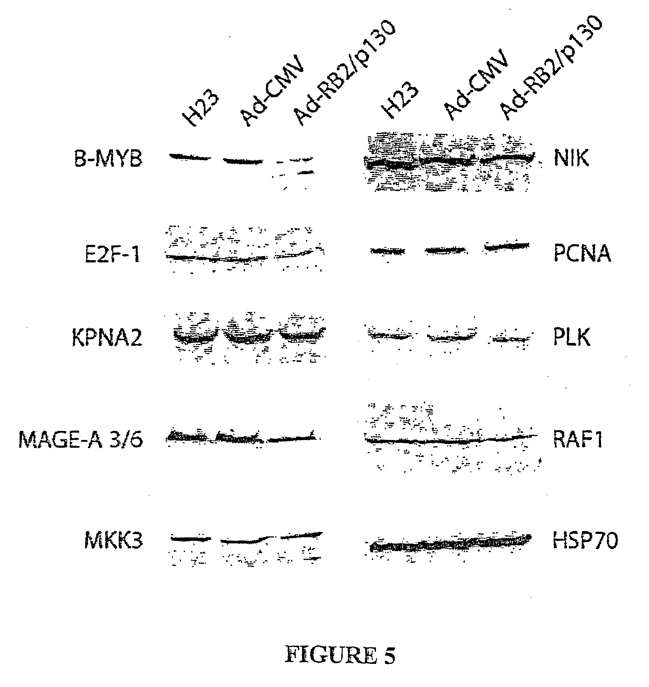

**Figure 5****.** Validation of oligonucleotide microarray data by western blot analysis. One hundred µg of protein extracts from H23, H23-Ad-CMV and H23-Ad-CMV-Rb2/p130 cells were loaded onto SDS-PAGE gels and immunoblotted with antibodies anti B-MYB, E2F-1, MAGEA 3/6, MKK3, NIK, PCNA, PLK and RAF1. Anti HSP-70 was used as internal control. The analysis was performed in duplicates with comparable results.

### DETAILED DESCRIPTION OF THE INVENTION

The present invention is based on the discovery of some of the molecular signatures or markers modulated by Rb2/p130 in non-small cell lung cancer (NSCLC) cells and the use of the molecular signatures as a basis for the administration of Rb2/p130 to modulate genes or gene expression patterns in the in non-small cell lung cancer cells.

Changes in cell phenotype in cancer are often the result of one or more changes in the gene expression of the cell. Some genes are expressed in tumor cells, and not in normal cells. In addition, different genes directly or indirectly induce cancer growth while others directly or indirectly suppress cancer growth. In fact, immunohistochemical analysis of the expression patterns of the Rb family members (pRb/p105, p107, and pRb2/p130) in 235 specimens of lung cancer (Baldi et al., 1996) and the expression pattern of pRb2/p130 in 158 specimens of human lung cancer showed an inverse correlation between the histological grading of the tumors, the development of metastasis, and the level of expression of pRb2/p130 (Baldi et al., 1997). A statistically significant inverse relationship between the histological grading and the expression of pRb/p105, p107 and pRb2/p130 was found in fine needle aspiration biopsies of squamous cell carcinoma patients (Minimo et al., 1999).

The invention described herein relates to the identification of a set of genes expressed in NSCLC cells that are modulated by Rb2/p130. In an aspect, the present invention simplifies prognosis determination by providing an identified set of genes whose expression in lung cancer cells can be modulated (down regulated or upregulated) which may predict clinical outcome as defined by, cell proliferation, tumor metastasis, recurrence, or death.

The protein and amino acid sequences of RB2/p130 and expression constructs of pRb2/p130 are known in the art (see, for example U.S. Patent 5,532,340). For example, to obtain expression constructs, a full length cDNA sequence of Rb2/p130 is subcloned into suitable retroviral or adenoviral vectors (MSCVneoEB ad MSCVPac) and such expression vectors are known to one skilled in the art.

In the present invention, RNA expression phenotyping was performed using high density oligonucleotide microarrays generated from quantitative expression data on over 3200 genes, which have been analyzed to identify specific genes down-regulated by RB2/p130 expression. The expression gene set can have several uses including, but not limited to, the following examples. The expression gene set may be used as a prognostic tool for lung cancer patients, to make possible more finely tuned diagnosis of lung cancer and allow physicians to tailor treatment to individual patients' needs. The invention can also assess the efficacy of NSCLC treatment by determining progression or regression of the lung cancer in patients before, during, and after the NSCLC cancer treatment. Another use of the expression gene set can be in the biotechnology and pharmaceutical industries' research on disease pathway discovery for therapeutic targeting. The invention can identify alterations in gene expression in lung cancer and can also be used to uncover and test candidate pharmaceutical agents to treat the lung cancer.

As used herein, a subject is a human although non-human mammals such as primate, rabbit, dog, cat, cow, horse, pig, sheep, goat and rodents are also contemplated. Preferably the subject is a human either suspected of having lung cancer or having been diagnosed with lung cancer. Methods for identifying subjects suspected of having lung cancer may include physical examination, subject's family medical history, subject's medical history, lung cancer biopsy, or a number of imaging technologies such as tomography ultrasound, magnetic resonance imaging, magnetic resonance spectroscopy, etc. Conventional diagnostic methods for lung cancer and the clinical delineation of lung cancer diagnoses are well known to those of skill in the medical arts.

As used herein, endometrial tissue sample is tissue obtained from an endometrial tissue biopsy using methods well known to those of ordinary skill in the related medical arts. In some instances a sample from the biopsy may be sufficient for assessment of RNA expression without amplification, but in other instances the lack of suitable cells in a small biopsy region may require use of RNA conversion and/or amplification methods or other methods to enhance resolution of the nucleic acid molecules. Such methods are well known to those of ordinary skill in the art and include, but are not limited to: direct RNA amplification, reverse transcription of RNA to cDNA (RT PCR), amplification of cDNA, or the generation of radio-labeled nucleic acids.

In the context of the present invention, "determining expression of gene or genes (or a set of nucleic acid molecules) in the lung cancer cells" means identifying RNA transcripts in the tissue sample by analysis of nucleic acid or protein expression in the tissue sample. As used herein, "set" refers to a group of genes classified in under a given category as listed in Table 2 herein. In some embodiments a set can include one or more categories or a combination of these categories.

The expression of the set of nucleic acid molecules in the sample from the lung cancer subject can be compared to the expression of the set of nucleic acid molecules in a sample of lung tissue that is non-cancerous. As used herein, non-cancerous lung tissue means tissue determined by one of ordinary skill in the medical art to have no evidence of lung cancer based on standard diagnostic methods including, but not limited to, histologic staining and microscopic analysis.

In the present invention, standard hybridization techniques of microarray technology are utilized to assess patterns of nucleic acid expression and identify nucleic acid marker expression. Microarray technology, which is also known by other names such as DNA chip technology and gene chip technology is well known to those of ordinary skill in the art and is based on, but not limited to, obtaining an array of identified nucleic acid probes on a fixed substrate, labeling target molecules with reporter molecules (e.g., radioactive, chemiluminescent, or fluorescent tags such as fluorescein, Cye3-dUTP, or Cye5-dUTP), hybridizing target nucleic acids to the probes, and evaluating target-probe hybridization. A probe with a nucleic acid sequence that perfectly matches the target sequence will, in general, result in detection of a stronger reporter-molecule signal than will probes with less perfect matches. Microarray technology is well known to one skilled in the art. The present invention also contemplates protein microarrays for analyzing molecular signatures in lung cancer cells or tissue.

In some instances, the micro array data can be validated using semi quantitative RT-PCR analysis, Northern blot analysis and/or Western blot analysis. These validation procedures are preferably used in instances where the determination of the gene expression level of specific pRb2/p130 target genes are desired.

### WORKING EXAMPLES

The following non-limiting examples and data are provided to illustrate various aspects and features relating to the methods of the present invention and as a further guide to one of ordinary skill in the art, and are not to be construed as limiting the invention in any way.

### Example 1: Effects of RB2/p130 adenoviral transduction on the H23 lung adenocarcinoma cell line

The human lung adenocarcinoma cell line H23 has been described previously (Claudio et al., 2000). The packaging cell line 293 (primary embryonal human kidney cells) transformed by sheared human adenovirus type 5 has been also previously described (Claudio et al., 1999). H23 cells were maintained in DMEM supplemented with 10% fetal bovine serum, 2 mM L-glutamine. 293 cells were maintained in DMEM supplemented with 10% heat inactivated fetal bovine serum, 2 mM L-glutamine.

Adenoviruses were generated by subcloning the full-length ORF of the *RB2*/*p130* gene into the pAd.CMV-Link1 vector to form the Ad.CMV-*RB2*/*p130* vims, as described previously (Claudio et al., 1999, Davis et al., 1998). The pAd.CMV-Link1 vector alone (to produce the Ad-CMV virus) was used as a negative control to assay the effects of viral infection alone without delivering a transgene. Adenoviruses were expanded, purified and tittered as previously described (Claudio et al., 1999).

Flow cytometry analysis (FACS) of exponentially growing H23 cells or H23 cells transduced with Ad-CMV or Ad-CMV-Rb2/p130 were carried out as previously described (Claudio et al., 1996). Briefly, 5X10⁵ cells were seeded and 24 hours after the cells were transduced with 50 MOI (multiplicity of infection) of adenoviruses. 48 hours after transduction, cells were collected and analyzed using a Coulter Flow cytometer.

For Northern blot analysis, H23 cells were grown to 60% confluency then infected with 50 MOI of adenoviruses carrying the *RB2*/*p130* gene or with the control Ad-CMV. After 14 h, medium was changed, and cells were harvested at 48 hours from the transduction. DNAse-treated total RNA from H23, H23-Ad-CMV and H23-Ad-CMV-Rb2/p130 transduced cells were extracted using TRIzol (Life Technologies, Inc, Grand Island, NY) according to manufacturer's protocol. Northern blot analysis was performed as previously described (Claudio et al., 1994).

Western blot analysis of exponentially growing H23 cells or of H23 cells transduced with Ad-CMV or Ad-CMV-Rb2/p130 were carried as previously described (Claudio et al., 1999). Extracts were normalized for protein content by Bradford analysis (Bio-Rad Laboratories, Inc., Melville, New York) and commasie blue gel staining. Primary anti-B-MYB, E2F-1, KPNA2, MKK3, NIK, PCNA, PLK, RAF1 (Santa Cruz Biotechnology, Inc., Santa Cruz, CA), anti-MAGE-A (Upstate, Lake Placid, NY) and anti-HSP70 (Oncogene Science, Cambridge, MA) were used following manufacturer's instructions.

H23 cells were plated at a density of 5x10⁵ in four 10-cm tissue culture dishes. Cell were transduced with 50 MOI of the control Ad-CMV or Ad-CMV-*RB2*/*p130* and harvested after 48 h. Two tissue culture dishes were used to extract mRNA. One tissue culture dish was used to extract the proteins and one for FACS analysis.

Northern blot analysis of samples transduced with *RB2*/*p130* showed an increased expression of the *RB2*/*p130* transcript more than 20 fold with respect to the control (Fig. 1a). Western blot analysis showed more than 100 fold enhanced expression of pRb2/p130 in the Ad-CMV-*RB2*/*p130* transduced cells (Fig. lb). To confirm the effects of pRb2/p130 enhanced expression in H23 cells we performed FACS analysis. Figure 2 shows that adenoviral Rb2/p130 transduction resulted in 81.2% of the cells accumulated in the G0/G1 phase of the cell cycle when compared to the control (54%).

### Example 2: Oligonucleotide microarray assay following enhanced expression of pRb2/p130 in a human lung adenocarcinoma cell line

Before submission of RNA samples for analysis protein extracts prepared from replicate plates of the corresponding cell culture were analyzed for expected enhanced expression of pRb2/p130 using western blots. Oligonucleotide-based microarrays were purchased from Mergen (Mergen Ltd. San Leandro, CA). ExpressChip H05000 DNA microarray system was used for this study. This array contains more than 3200 genes that are involved in a variety of different processes. DNase-treated total RNA (20µg) from H23 (parental cells), H23 cells transduced with Ad-CMV or Ad-CMV-RB2/p130 cell lines 48 hours after transduction were extracted using TRIzol (Life Technologies, Inc, Grand Island, NY) according to manufacturer's protocol. RNA integrity was verified for lack of degradation by formaldehyde gel electrophoresis. The biotin-labeled cRNA probes preparation, hybridization and array scanning were performed using Mergen Labeling/Hybridization/Detection Service.

Data acquisition and data analysis were performed using Imagene software (Biodiscovery Inc., Marina del Rey, CA) and Mergen's *Express*Data™ software (Mergen Ltd. San Leandro, CA). Briefly, data were processed for local background correction and normalization. Raw-Spot for each gene was calculated as the mean signal of the spot values minus that of local background. The Iₘₐₓ value was set to 65,000, after local background removal. A Normalization Coefficient (N) was applied to either the control population or to sample spot raw values to compensate for slide-to-slide and probe-to-probe variations. The Normalization coefficient was applied only if (Raw_spot/N) < Iₘₐₓ, otherwise values were set to Iₘₐₓ. Normalized values ≤0 were excluded from the analysis. Genes regulated by adenovirus transduction (Ad-CMV) with respect to the parental cell line were removed from the analysis. Spots with Mean Intensities > 45,000 were excluded for the ratio analysis. The expression ratios calculated with corrected values < mean of the local background on both channels were not used. Expression ratio of the analyzed genes were calculated comparing genes' expression values of H23 cells transduced with RB2/p130 with those of parental H23 or H23 cells transduced with Ad-CMV. A 2 fold or higher levels of target genes' expression ratio was considered significant, in accordance with most of the literature.

H23 cells were transduced with Ad-CMV or Ad-CMV-*RB2*/*p130.* Forty-eight hours later, 20 µg of DNA-free total RNA from H23, H23-Ad-CMV or H23-Ad-CMV-*RB2*/*p130* cells was reverse-transcribed and the double-strand cDNA was used as template to generate Cy3-labeled cRNA probes and then hybridized to the Mergen H05000 oligonucleotide-based microarray containing more than 3200 genes that are involved in a variety of different processes. Analysis was performed by Mergen Ltd, San Leandro, CA. Microarray experiments were performed comparing H23 vs. H23-Ad-CMV; H23-Ad-CMV vs. H23-Ad-CMV-*RB2*/*p130*; and H23 vs. H23-Ad-CMV-*RB2*/*p130* cells. Duplicate experiments were carried out on a single total RNA preparation from the cells.
In this study 40 genes were downregulated more than 2.0-fold (Table 1). Figure 3 shows the plots of the differential expression of 3263 genes in H23-Ad-CMV vs. H23-Ad-CMV-*RB2*/*p130* cells and H23 vs. H23-Ad-CMV-*RB2*/*p130* cells. Overall, the expression of the majority of the spotted genes was not altered by *RB2*/*p130.* Modulated genes were classified in table 2 on the basis of a well documented and established biological or pathological function of the encoded protein. The genes downregulated by pRb2/p130 enhanced expression belong mainly to the following categories: cell division, signaling and communication, cell structure and motility, gene expression, metabolism, and disease.

### Example 3: Validation of the oligonucleotide microarray assay using semi quantitative RT-PCR and western blot analysis..

RT-PCR was used to analyze target gene expression in the present study. A 2 µg aliquot of DNAse-treated total DNA from each sample was reverse-transcribed for single stranded cDNAs using M-MLV reverse transcriptase (Invitrogen, Carlsbad, CA) according to manufacturer's protocol. The same cDNA product obtained from each sample was used for subsequent PCR amplification with the primer sets prepared for the target gene and β-*actin* (Act-β)/*HPRT* housekeeping genes. The amplification of the β-*actin* and *HPRT* genes were used as double internal control. Ratio between the samples and each housekeeping gene was calculated to normalize for initial variations in sample concentration and as control for reaction efficiency. Primer sequences were designed using the software Primer 3 (developed by Steve Rozen, Helen J. Shaletsky) available on-line at http://www-genome.wi.mit.edu. Primer sequences can be provided upon request. PCR reaction conditions were individually optimized for each gene product studied and the number of PCR cycles was setup to be within the linear range of product amplification.

In each experiment, possible DNA contamination was determined by a control reaction in which reverse transcriptase was omitted from the reaction mixture. PCR products were loaded onto ethidium bromide stained 1.5% agarose gels. Densitometric analysis of the PCR products were performed using an Alpha Imager system (Alpha Innotech Corporation, San Leandro, CA) and the ImageJ v1.29 software (developed by Wayne Rasband) available on-line at http://rsb.info.nih.gov/ij/. All PCR products were purified using QIAquick PCR purification kit (Qiagen, Santa Clarita, CA) and their identities verified by automated DNA forward and reverse sequencing using a dideoxy terminator reaction chemistry for sequence analysis on the Applied biosystem Model 373A DNA sequencer.

*E. coli* dH5alpha cells transformed with recLic B-GFP constructs were cultured or fermented by overnight culturing process in LB media. The fermentation was continued for12 h and harvested at a cell density of10⁴. Two liters of cell culture or fermentation broth were divided into 1 liter containers/ /bottles and centrifuged at 10,000 rpm for 30 min in a centrifuge. The supernatant was discarded and the pellet was used to recover the carrier protein.

To determine the gene expression level of specific pRb2/ p130 target genes, semiquantitative RT-PCR analysis was used. A panel of 11 genes, randomly selected among the 40 identified by microarray analysis, was analysed. We confirmed this by RT-PCR downregulation of BMYB, Cyc B2, Cyc D1, GRPR, KPNA2, MKK3, NIK, PCNA, PIM, PLK, and RAF-1 (Figure 4). Genes highly downregulated (range between 6-and 17-fold) in microarray analysis such as PCNA, MKK3, B-MYB, and NIK showed a comparative downregulation in semiquantitative RT-PCR analysis between 7-and 3.5-fold. Genes still downregulated in microarray analysis, but at a lower extent such as RAF-1, PIM1, CycD1, GRPR, KPNA2, and CycB2, showed a comparable downregulation in semiquantitative RT-PCR analysis between 3.4-and 2.0-fold. PLK, which showed a high downregulation ratio in microarray analysis, failed to be validated by semiquantitative RT-PCR. In fact, PLK showed almost a two-fold difference expression level by RT-PCR. Of the 11 transcriptionally downregulated genes that were studied by RT-PCR analysis, only seven genes (B-MYB, KPNA2, MKK3, NIK, PLK, and RAF-1) were found expressed by Western blot analysis at a lower level upon enhanced pRb2/p130 expression with a ratio between 1.9-and 3.0-fold (Figure 5). As the MAGE gene family is composed of 23 related genes divided into four clusters and the MAGE-A subfamily comprises 12 genes highly identical in their coding sequence, we were not able to perform RT-PCR on this gene family, but we could confirm by Western blot analysis the contingent downregulation of MAGEA-3/6 to enhanced pRb2/p130 expression. Surprisingly, PCNA that was highly down-regulated in the microarray analysis, also appearing modulated in RT-PCR, showed no protein expression changes upon enhanced pRb2/p130 expression. However, it has been shown that PCNA has a relatively long half-life that can extend beyond the S phase into the M phase and beyond into the G0 phase of cells in rapidly proliferating tumors.

All publications, patents and patent applications mentioned in the specification are indicative of the level of those skilled in the art to which this invention pertains. All publications, patents and patent applications referred to herein are incorporated herein by reference to the same extent as if each individual publication or patent application was specifically and individually indicated to be incorporated by reference. While this invention has been described with a reference to specific embodiments, it will be obvious to those of ordinary skill in the art that variations in these methods and compositions may be used and that it is intended that the invention may be practiced otherwise than as specifically described herein. Accordingly, this invention includes all modifications encompassed within the spirit and scope of the invention as defined by the claims.

**Table 1 Downregulated genes by RB2/p130 adenovirus-enhanced expression**

| | *GenBankTM* | *ID Gene description* | *Gene symbol* | *Ratio 1* | *Ratio 2* | *Average* |
|---|---|---|---|---|---|---|
| 1 | M15796 | Proliferating cell nuclear antigen | PCNA | 16.4 | 17.5 | 16.9 |
| 2 | L36719 | Mitogen-activated protein kinase kinase 3 | MKK3 | 12.4 | 19.2 | 15.8 |
| 3 | X13293 | V-myb avian myeloblastosis viral oncogene homolog-like 2 | B-MYB | 8.2 | 13 | 10.6 |
| 4 | Z36714 | Cyclin F | CCNF | 6.6 | 8.7 | 7.6 |
| 5 | AF053306 | Budding uninhibited by benzimidazoles 1 (yeast homolog), β | BUBIB | 8.9 | 6 | 7.4 |
| 6 | L19559 | Polo (Drosophia)-like kinase | PLK | 6.5 | 7.5 | 7 |
| 7 | Y10256 | Mitogen-activated protein kinase kinase kinase kinase 4 | NIK | 5.6 | 7.6 | 6.6 |
| 8 | D14678 | Kinesin-like 2 | KNSL2 | 5 | 6.7 | 5.8 |
| 9 | U33849 | Proprotein convertase subtilisin/kexin type 7 | PCSK7 | 4.4 | 6.5 | 5.4 |
| 10 | AF002822 | Cyclin B2 | CCNB2 | 5.3 | 4.2 | 4.7 |
| 11 | L16862 | G protein-coupled receptor kinase 6 | GPRK6 | 3.5 | 5.7 | 4.6 |
| 12 | L20010 | Host cell factor C1 | HCFC1 | 3.2 | 5.9 | 4.5 |
| 13 | AB002359 | FGAR amidotransferase | PFAS | 3.7 | 5 | 4.3 |
| 14 | X63692 | DNA (cytosine-5-)-methyltransferase 1 | DNMT1 | 3.5 | 4.9 | 4.2 |
| 15 | U09559 | Karyopherin α 2 | KPNA2 | 3.5 | 4.1 | 3.8 |
| 16 | AF011468 | Serine/threonine kinase 15 | STK15 | 3.7 | 4 | 3.8 |
| 17 | U21847 | TGFB-inducible early growth response | TIEG | 3.3 | 4.3 | 3.8 |
| 18 | F046078 | Budding uninhibited by benzimidazoles 1 (yeast homolog) | BUB1 | 4.3 | 3.1 | 3.7 |
| 19 | M25269 | ELK1, member of ETS oncogene family | ELK1 | 3.1 | 3.1 | 3.1 |
| 20 | D78335 | Uridine monophosphate kinase | UMPK | 3.2 | 3.1 | 3.1 |
| 21 | X51804 | Putative receptor protein | PMI | 2.4 | 3.7 | 3 |
| 22 | AB018330 | Calcium/calmodulin-dependent protein kinase kinase 2, β | CAMKK2 | 2.1 | 3.7 | 2.9 |
| 23 | L33801 | Glycogen synthase kinase 3 β | GSK3B | 3.2 | 2.6 | 2.9 |
| 24 | AF001903 | L-3-hydroxyacyl-Coenzyme A dehydrogenase, short chain | HADHSC | 3.2 | 2.6 | 2.9 |
| 25 | M81735 | Polymerase (DNA directed), δ 1, catalytic subunit | POLD1 | 2.5 | 3.3 | 2.9 |
| 26 | M32110 | Nucleolar protein I | NOL1 | 2.7 | 3 | 2.8 |
| 27 | X97630 | ELKL motif kinase | EMK1 | 2.4 | 3.3 | 2.8 |
| 28 | M73481 | Gastrin-releasing peptide receptor | GRP-R | 2.7 | 2.7 | 2.7 |
| 29 | AF035586 | X-ray repair complementing defective repair in Chinese hamster cells 3 | XRCC3 | 2.5 | 3 | 2.7 |
| 30 | D10704 | Choline kinase | CHK | 3 | 2.3 | 2.6 |
| 31 | U10339 | Melanoma antigen, family A,3/6 | MAGE-A3/6 | 2.1 | 3 | 2.5 |
| 32 | Y13936 | Protein phosphatase IG (formerly 2C), magnesium-dependent, gamma isoform | PPM1G | 2.3 | 2.8 | 2.5 |
| 33 | AB000509 | TNF receptor-associated factor 5 | TRAF5 | 2.2 | 2.7 | 2.4 |
| 34 | AJ005016 | ATP-binding cassette, sub-family F (GCN20), member 2 | ABCF2 | 2.4 | 2.5 | 2.4 |
| 35 | U6382 | TEA domain family member 4 | TEAD4 | 2.5 | 2.2 | 2.3 |
| 36 | M54915 | Pim-I oncogene | PIM1 | 2.2 | 2.3 | 2.2 |
| 37 | X59798 | Cyclin D1 | CCND1 | 2.1 | 2.1 | 2.1 |
| 38 | M63256 | Cerebellar degeneration-related protein | CDR2 | 2.2 | 2.1 | 2.1 |
| 39 | D26599 | Proteasome subunit, β type, 2 | PSMB2 | 2.2 | 2.1 | 2.1 |
| 40 | X03484 | V-raf-1 murine leukemia viral oncogene homolog 1 | RAF1 | 2.3 | 2 | 2.1 |

| | | | | | | |
|---|---|---|---|---|---|---|
| Genes that are downregulated more than 2.0-fold in response to the enhanced expression of RB2/p130 by microarray analysis are listed. Genes were identified as unique as mentioned in the GenBank™ and are sorted in descending order. Ratio 1 indicates the fold of repression for each gene as determined by microarray analysis of H23-Ad-CMV *vs* H23·Ad-CMV-RB2/p130. Ratio 2 indicates the fold of repression for each gene as determined by microarray analysis of H23 *vs* H23-Ad-CMV-RB2/p130 | | | | | | |

**Table 2 Classification of RB2/p130-repressed genes by category**

| *Category* | *Genes* |
|---|---|
| ATPase/GTPase/ATP binding/GTP binding | ABCF2 |
| | KNS L2 |
| | |
| Calcium/potassium/sodium/iron binding protein | CAMKK2 |
| | |
| Cell cycle/cyclins | BUB1 |
| | BUB1B |
| | CCNB1 |
| | CCNB2 |
| | CCND1 |
| | CCNF |
| | B-MYB |
| | NOL1 |
| | PCNA |
| | PLK |
| | PPM1G |
| | |
| Cell surface/antigen | ABCF2 |
| | CDR2 |
| | GPRK6 |
| | GRP-R |
| | KNSL2 |
| | MAGE-A 3/6 |
| | PCNA |
| | PMI |
| | |
| Chromosome/chromatin/histone | DNMT1 |
| | PLK |
| | XRCC3 |
| | |
| Cytokines and growth factors | GRP-R |
| | PCSK7 |
| | TIEG |
| | TRAF5 |
| | |
| Cytoskeleton/microtubules/microfilaments/motility | CAMKK2 |
| | EMK1 |
| | KNSL2 |
| | |
| Differentiation/development | BUB1 |
| | GSK3B |
| | B-MYB |
| | NOL1 |
| | PIM1 |
| | PLK |
| | RAF1 |
| | TEAD4 |
| | TIEG |
| | |
| Diseases | B-MYB |
| | CCND1 |
| | CDR2 |
| | ELK1 |
| | MAGE-A 3/6 |
| | NOL1 |
| | PCSK7 |
| | PIM1 |
| | RAF1 |
| | STK15 |
| | |
| DNA binding/damage/recombination | DNMT1 |
| | POLD1 |
| | XRCC3 |
| | |
| G protein/regulators of G protein signaling | GPRK6 |
| | GRP-R |
| | PMI |
| | |
| Hydrolase/hydrolysis/hydrolyses | ABCF2 |
| | PCSK7 |
| | PPM1G |
| Kinases | BUB1 |
| | BUBIB |
| | CAMKK2 |
| | CHK |
| | ELK1 |
| | EMK1 |
| | GPRK6 |
| | GSK3B |
| | MKK3 |
| | NIK |
| | PLK |
| | RAF1 |
| | STK15 |
| | UMPK |
| | |
| Lipoproteins/tipids , | CHK |
| | |
| Membrane trafficking | DNMT1 |
| | KPNA2 |
| | |
| Mitochondrial proteins | ABCF2 |
| | HADHSC |
| | |
| Nuclear receptors/receptors | BUB1 |
| | DNMT1 |
| | ELK1 |
| | GPRK6 |
| | GRP-R |
| | NOL1 |
| | PCNA |
| | PMI |
| | POLD1 |
| | PPMIG |
| | TIEG |
| | TRAF5 |
| | |
| Oncogenes | B-MYB |
| | ELK1 |
| | PIM1 |
| | RAF1 |
| | |
| Phosphatase/proteases/peptidase | PCSK7 |
| | PPMIG |
| | PSMB2 |
| | |
| Signal transduction | CAMKK2 |
| | GPRK6 |
| | GRP-R |
| | MKK3 |
| | NIK |
| | PMI |
| | TRAF5 |
| | |
| Synthetase/synthase | GSK3B |
| | PFAS |
| | |
| Transcription/transcription factor | B-MYB |
| | CDR2 |
| | ELK1 |
| | HCFC1 |
| | TEAD4 |
| | TIEG |
| | |
| Transporters | ABCF2 |
| Transferases | DNMT1 |
| | PFAS |

| | |
|---|---|
| The analysed genes are classified on the basis of established biological or pathological functions of the encoded proteins. Genes that are listed in one category are indicated in bold | |

The present invention furthermore relates to the following items:
1. A method of determining whether to use a RB2/p130 gene expression system or a protein encoded by the system to modulate a gene or gene expression pattern in lung cancer cells of a human test subject, the method comprising:
   providing molecular signatures modulated by RB2/p130 for lung cancer cells, wherein the molecular signatures comprise expression products of at least one of the genes selected from the group consisting of: PCNA, MKK3, B-MYB, CCNF, BUB1B,PLK, NIK, KNSL2, PCSK7, CCNB2, GPRK6, HCFC1, PFAS, DNMT1, KPNA2, STK15, TIEG, BUB1 ELK1, UMPK, PMI, CAMKK2, GSK3B, HADHSC, POLD1, NOL1, EMK1, GRP-R, XRCC3, CHK, MAGEA3/6, PPM1G, TRAF5, ABCF2, TEAD4, PIM1, CCND1, CDR2, PSMB2 and RAF1;
   determining gene or genes expressed in the lung cancer cells of the human test subject; and
   using the RB2/p130 gene expression system or the protein to modulate the gene or the gene expression pattern in the lung cancer cells of the human test subject if it is determined that the gene or genes expressed in the lung cancer cells of the human test subject are the same as the at least one of the genes.
2. The method of item 1 wherein the genes selected are B-MYB, PCSK7, STK15, ELK1, NOL1, MAGEA3/6, PIM1, CCND1, CDR2, and RAF1.
3. The method of item 1, wherein the human test subject is post-treatment for a non-small cell lung cancer.
4. The method of item 3, wherein the treatment is selected from the group consisting of surgical operation, chemotherapy, radiation therapy and RB2/p130 gene therapy or combinations thereof.

## Claims

1. A method for diagnosing cancer in a human tissue, comprising
(i) measuring the expression level of one or more genes selected from the group consisting of: PCNA, MKK3, B-MYB, CCNF, BUB1B, PLK, NIK, KNSL2, PCSK7, CCNB2, GPRK6, HCFC1, PFAS, DNMT1, KPNA2, STK15, TIEG, BUB1, ELK1, UMPK, PMI, CAMKK2, GSK3B , HADHSC, POLD1, NOL1, EMK1, GRP-R, XRCC3, CHK, MAGEA3/6, PPM1G, TRAF5, ABCF2, TEAD4, PIM1, CCND1, CDR2, PSMB2 and RAF1, in cells of said tissue obtained from a human test subject, and
(ii) comparing the expression level determined in (i) with the expression level of said one or more genes after transformation of cells of said tissue obtained from a human test subject with the RB2/p130 gene by means of an adenoviral vector, wherein a down-regulation of the expression level after transforming the cells indicates a diagnosis of cancer in the tissue.

2. The method of claim 1, wherein the genes are selected from the group consisting of: B-MYB, PCSK7, STK15, ELK1, NOL1, MAGEA3/6, PIM1, CCND1, CDR2 and RAF1.

3. The method of claim 1 or 2 wherein the cancer is non-small cell lung cancer.

4. The method of any one of claims 1 to 3 wherein the expression level of the one or more genes is measured using an oligonucleotide or protein microarray assay.

5. A method for diagnosing cancer in a human tissue, comprising
(i) measuring the expression level of one or more genes selected from the group consisting of: PCNA, MKK3, B-MYB, CCNF, BUB1B, PLK, NIK, KNSL2, PCSK7, CCNB2, GPRK6, HCFC1, PFAS, DNMT1, KPNA2, STK15, TIEG, BUB1, ELK1, UMPK, PMI, CAMKK2, GSK3B , HADHSC, POLD1, NOL1, EMK1, GRP-R, XRCC3, CHK, MAGEA3/6, PPM1G, TRAF5, ABCF2, TEAD4, PIM1, CCND1, CDR2, PSMB2 and RAF1 in cells of said tissue obtained from a human test subject, and
(ii) comparing the expression level determined in (i) with the expression level of said one or more genes after transformation of cells of said tissue obtained from a human test subject with RB2/p130;
wherein a down-regulation in the expression level after treating the cells with RB2/p130 indicates a diagnosis of cancer in the tissue.

6. The method of claim 5, wherein the genes are selected from the group consisting of: B-MYB, PCSK7, STK15, ELK1, NOL1, MAGEA3/6, PIM1, CCND1, CDR2 and RAF1.

7. The method of claim 5 or 6 wherein the cancer is non-small cell lung cancer.

8. The method of any one of claims 5 to 7 wherein the expression level of the one or more genes is measured using an oligonucleotide or protein microarray assay.
